Europäisches Patentamt

European Patent Office

Office européen des brevets

(19)

(11) **EP 1 455 009 A1**

(12) **EUROPÄISCHE PATENTANMELDUNG**

(43) Veröffentlichungstag:
**08.09.2004  Patentblatt 2004/37**

(51) Int Cl.⁷: **D03D 15/00**, A41D 31/00,
A61N 5/00

(21) Anmeldenummer: 04405109.2

(22) Anmeldetag: **26.02.2004**

(84) Benannte Vertragsstaaten:
**AT BE BG CH CY CZ DE DK EE ES FI FR GB GR
HU IE IT LI LU MC NL PT RO SE SI SK TR**
Benannte Erstreckungsstaaten:
**AL HR LT LV MK**

(30) Priorität: **04.03.2003  CH 3322003**

(71) Anmelder: **VIP Domotec S.A.R.L.
3364 Leudelange (LU)**

(72) Erfinder: **Hahn, Franz-Josef
56235 Ransbach-Baumbach (DE)**

(74) Vertreter: **Gaggini, Carlo, Dipl.Ing.
Brevetti-Marchi
Via ai Campi 6
6982 Agno (CH)**

(54)    **Schutz-Gewebe bestehend aus mindestens zwei Fasertypen mit verschiedenen
physikalischen Eigenschaften**

(57)    Die vorliegende Erfindung betrifft ein Schutzgewebe, das aus mindestens zwei Fasertypen mit unterschiedlichen physikalischen Eigenschaften besteht.

Das Ziel der vorliegenden Erfindung ist, ein Gewebe vorzuschlagen, das die Schutzeigenschaften bestimmter Fasern gegen elektrostatische Felder und elektromagnetische Felder mit den günstigen Eigenschaften von Fasern in sich vereinigt, die "biologische" Infrarotstrahlen ausstrahlen.

Diese Ziele werden erreicht, indem beispielsweise die Fasern mit Biokeramikmaterial vereint werden mit Fasern, die einen Abschirmeffekt gegen elektrostatische Felder und elektromagnetische Felder bewirken, insbesondere Kohlefasern.

Das mit der erfindungsgemässen Kombination von Fasern hergestellte Gewebe wird vorzugsweise verwendet zur Herstellung von Schutzabdeckungen für einzelne Körperteile (beispielsweise Knieschonern, Ellbogenüberzüge, usw.) sowie zur Herstellung von Dekken, Überwürfen, usw.

fig 1/a

EP 1 455 009 A1

Figuren 1.a und 1b: Vergleich der Leistungsspektren des schwarzen Körpers (obere Kurve) und des Mustergewebes (untere Kurve)

## Beschreibung

Die vorliegende Erfindung betrifft ein Schutz-Gewebe bzw. textiles Flächengebilde bestehend aus mindestens zwei Fasertypen mit verschiedenen physikalischen Eigenschaften gemäss dem Oberbegriff des Anspruchs 1.

Gemäss einer bevorzugten Ausführungsform der vorliegenden Erfindung enthält das Gewebe zugleich eine Komponente von Fasern mit biokeramischem Material zur Rückstrahlung des vom menschlichen Körper ausgestrahlten Infrarots sowie eine Komponente von Fasern, die einen Abschirmungseffekt gegen statische Felder und elektromagnetische Felder ausüben.

Gegenstand der vorliegenden Erfindung ist daher vorzugsweise die Zuordnung von biokeramischen Fasern und von Kohlefasern in einem einzigen Gewebe mit dem Zweck, eine innovative Wirksamkeit in der Kombination der beiden Basis-Komponenten im Gewebe zu erreichen.

Im Folgenden werden daher zuerst die einzelnen Wirkungen der zwei Komponenten analysiert, wobei die Eigenschaften von Geweben, in denen nur an sich bekanntes Biokeramikmaterial vorhanden ist, in Betracht gezogen werden, sowie jene von Geweben, in denen nur an sich auch bekannte Kohlefasern vorhanden sind.

## Biokeramik-Material im Gewebe

## Langwelliges Infrarot (FIR = Far Infrared Radiation)

Alle Gegenstände, deren Temperatur über dem absoluten Nullpunkt (-273°C) liegt, strahlen thermische Energie in Form von Infrarot-Strahlen ab.

Im Fall des menschlichen Körpers beträgt die mittlere Oberflächentemperatur 34°C, was einer Abstrahlung von langwelligem Infrarot (FIR) entspricht. Diese Strahlung bildet das Emissionsspektrum des menschlichen Körpers im Wellenlängenbereich von 8 bis 15 Mikron.

Bei Bestrahlung mit FIR-Wellen mit Wellenlängen, die der vom menschlichen Körper natürlicherweise abgestrahlten Strahlung ähnlich sind, ergibt sich eine harmonisierende Wirkung auf die organische Substanz, die den grössten Teil des Menschen ausmacht, nämlich das Wasser.

Wasser ist das hauptsächlichste Element, aus dem die Lebewesen bestehen, mit 60 bis 70% des Körpergewichts. Es ist im Blut vorhanden, in den Lymphgefässen, in den Zell-Zwischenräumen sowie in den Zellen selbst.

Zwei grosse Flüssigkeitsbereiche lassen sich unterscheiden, ein Bereich innerhalb der Zellen (50% des Körpergewichts) und ein Bereich ausserhalb der Zellen (20% des Körpergewichts, wovon 15% in den Zwischenräumen und 5% Plasma). Diese beiden Bereiche sind durch Zellmembranen voneinander getrennt, die eine selektive Permeabilität aufweisen, und die Transporte von Wasser und Elektrolyten ausgleichend harmonisieren.

Die Wassertransporte im Organismus sind von grundlegender Bedeutung für das Aufrechterhalten der biologischen Prozesse, insbesondere für die Erneuerung der Haut und der subkutanen Gewebe. Falls dieses Gleichgewicht gestört ist, zeigen sich als Signal zuerst oft eine wässerige Stauungen in der Haut, mit Drainage-Problemen und Ödemen.

Unser Organismus ist in seinem Gleichgewichtszustand fähig, die erforderliche Energie zur Bildung von freiem Wasser aufzubringen, doch diese Energie kann sich abschwächen, und in jedem Fall schwindet sie mit zunehmendem Alter. Die Anwendung von "biologischer" Infrarotstrahlung (mittlere bis lange Infrarotwellen) ermöglichen die Versorgung der tiefer liegenden Gewebe durch die Haut mit einer sanften Energie, die dazu beiträgt, die natürlichen Prozesse zu stimulieren, insbesondere die mit Wasser verbundenen, dank einer Art "Resonanz-Absorption"; aus diesen Phänomenen resultiert eine bessere Drainage der Gewebe, bessere Ausscheidung der Toxine und eine bessere Sauerstoffversorgung.

Sich dem Sonnenlicht aussetzen erlaubt eine Aufnahme von etwa 20% an Infrarotstrahlung. Menschen sind jedoch nicht ständig an der Sonne. Zudem strahlt die Sonne auch Strahlen aus, die der menschlichen Gesundheit schaden können.

Es wurde eine bestimmte Keramik-Kombination gefunden, allgemein "Biokeramik" genannt, die sich bestens dazu eignet, "biologische" Infrarotstrahlung abzugeben, also mit Wellenlängen "λ" im Bereich von 6 bis 14 μm.

Die Keramikpulver werden durch Mischung herkömmlichen Keramikmaterials mit verschiedenen Oxydmaterialien bei sehr hoher Temperatur mittels raffinierter Verfahren hergestellt. Die meistgebrauchten Mineraloxyde sind Siliziumoxyd ($SIO_2$), Aluminiumoxyd ($AL_2O_2$), Magnesiumoxyd ($MgO$), Titanoxyd ($TiO_2$).

Als "Biokeramik" werden auch Keramikmaterialien natürlichen Ursprungs verwendet, wie beispielsweise Zeolite, deren physikalisch-chemische Eigenschaften überprüft worden sind.

Die Verwendung von Biokeramik-Materialien, die Infrarot-Strahlung mit Wellenlängen von 6 bis 14 Mikron abgeben, trägt bei Abstrahlung direkt im Kontakt mit der Haut dazu bei, die Homöostase der Gewebe zu verstärken. Die wesentlichste Wirkung besteht in der besonderen Aktivierung der Wassermoleküle, mit günstigen Wirkungen auf die Regulierung des Wasseraustausches zwischen den verschiedenen Flüssigkeits-Bereichen.

Diese Wirkung verbessert den Austausch im Gewebe, die Zufuhr von Sauerstoff und Nährstoffen, den Abtransport von Abfallstoffen, Toxinen und Kohlenstoff-Anhydrid. Alle diese günstigen Einflüsse gehen direkt oder indirekt auf diese Wirkungen zurück.

**Biokeramik als "Schwarzer Körper" mit langwelliger Infrarotstrahlung**

Alle in den hier interessierenden Geweben verwendeten Biokeramikmaterialien weisen eine gemeinsame Eigenschaft auf: Ihr Emissionsspektrum entspricht etwa jenem eines schwarzen Körpers, und die Strahlungsleistung weist zwischen 4 und 16 μm etwa bei 10 μm ihr Maximum auf.

Im Sinn eines Beispiels sei hier aufgezeigt, wie sich ein Gewebe verhält, dessen Verbrennungsrückstand die folgende chemische Zusammensetzung aufweist:

| | |
|---|---|
| $Al_2O_3$ | 0.03 |
| $F_2O_3$ | <0.01 |
| $TiO_2$ | <0.01 |
| CaO | 0.02 |
| MgO | <0.01 |
| $Na_2O$ | 0.01 |
| $K_2O$ | <0.01 |
| $SiO_2$ | 0.83 |

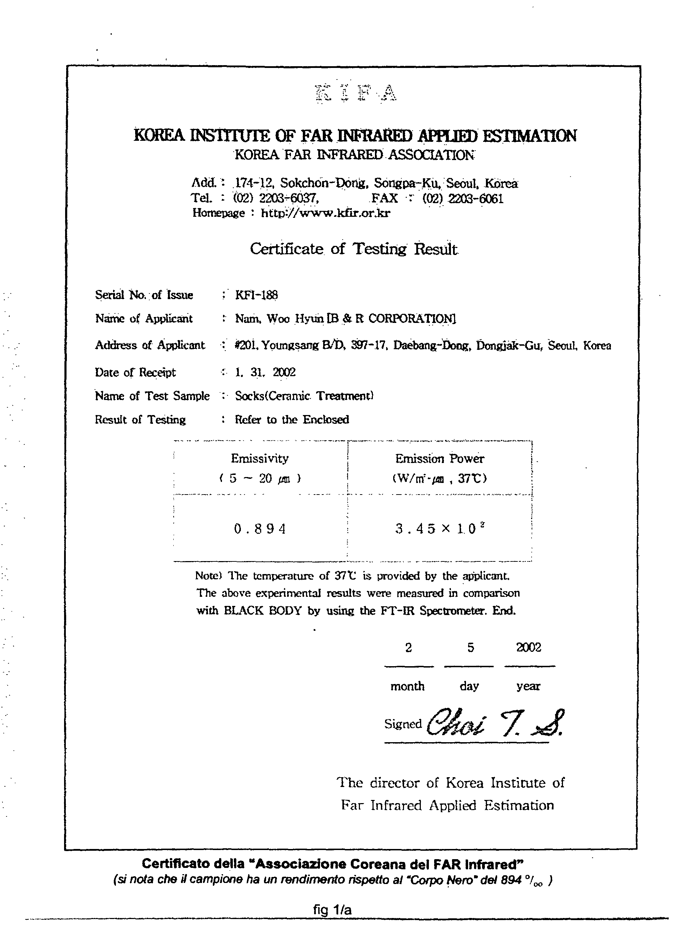

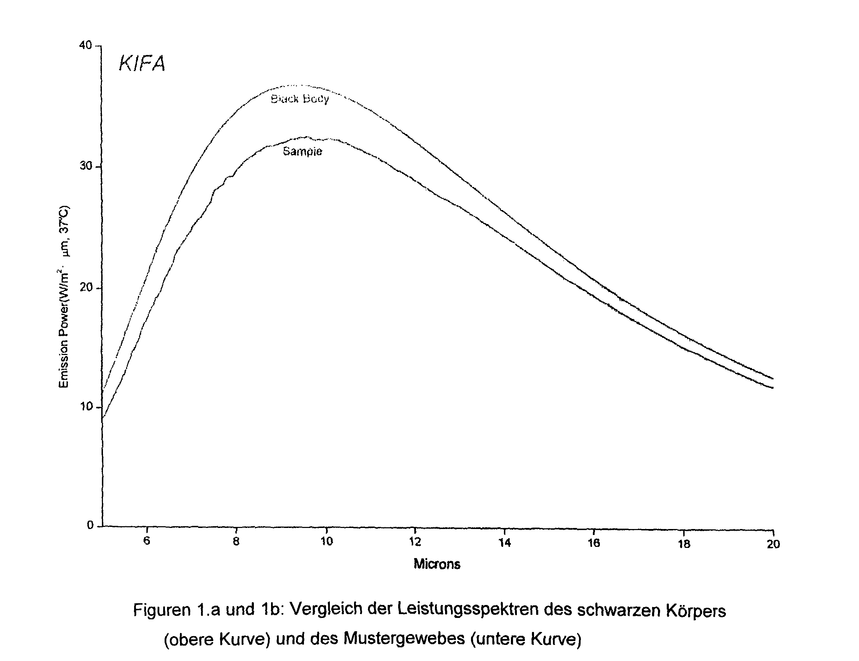

In den Figuren 1a und 1b sind die Emissionsspektren des gewählten Musters und eines schwarzen Körpers bei einer Temperatur von 310 K, also 37°C, einander gegenübergestellt.

Die theoretische Spektralkurve entspricht der folgenden Gleichung:

$$R_\lambda = \frac{2c^2h}{\lambda^5} \times \frac{1}{e^{hc/\lambda KT} - 1}$$

wobei:

R = Strahlungsleistung
T=°K=273+°C
c = 2.997930 x $10^8$ m/sec
h = 6.6252 $10^{-34}$ Joule/sec
k = 1.38 x $10^{-23}$ Joule/°K

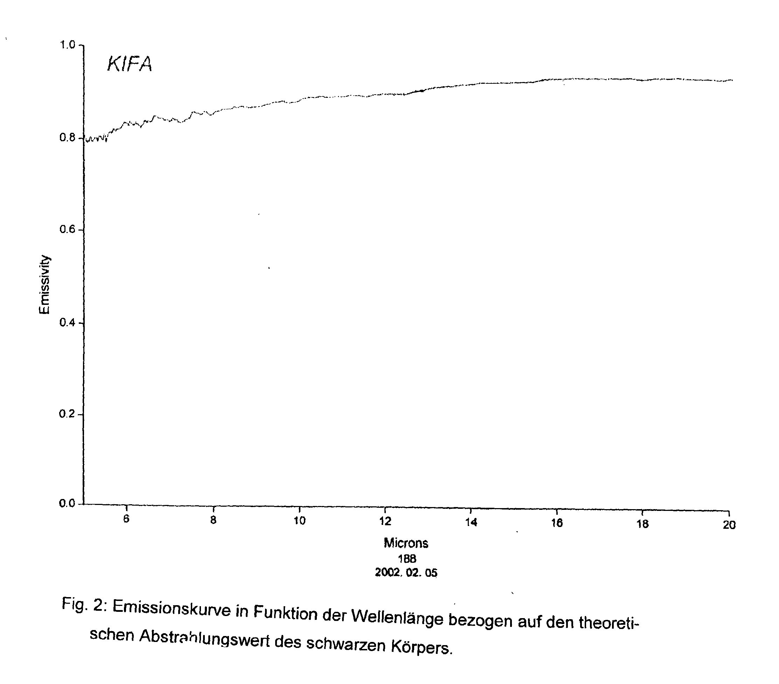

Die totale Strahlungsleistung des Mustergewebes beträgt, wie aus der Fig. 2 ersichtlich ist, 89.4% der Strahlungsleistung des schwarzen Körpers bei $T_i$ = 310 K (273 + 37°C), wobei sich die bei der Temperatur $T_i$ abgestrahlte Leistung gemäss dem Gesetz von Stephan-Boltzmann errechnet:

$$P_i = 0.894 \times \left[ \int_{\lambda=0}^{+\infty} \frac{2\pi c^2h}{\lambda^5} \times \frac{1}{e^{hc/\lambda KT_i} - 1} \right] = 3.45 \times 10^2 \text{ Watt/m}^2$$

wobei:

$P_i$ = Totale Strahlungsleistung bei der Temperatur $T_i$, in Watt/m$^2$
T =°K=273+37
c = konstant = 5.78 x $10^{-8}$ Watt/m$^2$ x °K$^4$

Über die quantitativen Aspekte hinaus ist von Interesse festzustellen, wie das biokeramische Material bei einer Temperatur, die der Körpertemperatur des Menschen entspricht oder nahe kommt, eine Strahlung abgibt, deren Spektrum genau jenem des langwelligen Infrarot (FIR) entspricht und sich daher gegenüber dem vom menschlichen Körper abgestrahlten Infrarot wie ein Spiegel verhält, wobei in Wirklichkeit die Infrarotstrahlung absorbiert und auf den gleichen Wellenlängen wiederum gegen den Körper hin abgestrahlt wird.

**Kohlefasern im Gewebe**

**Vorbemerkung - Mögliche Schäden im menschlichen Körper durch elektrische und magnetische Felder**

An dieser Stelle ist nicht beabsichtigt, auf die Gefährlichkeit oder den Grad der Gefährdung bei Exposition des Körpers in elektromagnetischen Feldern oder in elektrostatischen Feldern zu diskutieren.

Unter den vielen möglichen zugeschriebenen Nachteilen und Gefahren seien hier lediglich einige Angaben als Resultat der Studie "Society of Hyperthermic Oncology" - Japan und "Washington Education" erwähnt (www.ee. washington.edu/coselec/CE/sp95reports/espinosa/body3.htm - 5k).

In verschiedenen Laboratorien wurden menschliche Brustkrebszellen einer Melatonin-Infusion ausgesetzt, das ein natürliches Neuro-Hormon ist, das wir alle besitzen, und das uns während der Nacht richtig zu schlafen hilft. Melatonin übt einen wirksamen onkostatischen Einfluss aus und wirkt, anders ausgedrückt, gegen die Bildung und Vermehrung von Tumorzellen.

Solche Zellen wurden sodann einem elektrischen Wechselfeld von 50 HZ ausgesetzt, und dabei konnte festgestellt werden, dass der onkostatische Einfluss des Melatonins verschwindet.

Während der Nacht, wenn wir schlafen gehen, steigt der Melatonin-Spiegel an, und das Melatonin wird im Blutstrom durch unsere Zellen transportiert, wo es eine eigentliche "Putzarbeit" verrichtet und in der Tat einen wirksamen Einfluss auf die Ausschaltung der freien Radikale ausübt.

Das Verweilen freier Radikale in unserem Organismus ruft Schädigungen an der DNA der Zellen hervor wobei sie die Wahrscheinlichkeit der Bildung von Krebszellen erhöhen.

Melatonin ist ein Wirkstoff, der besonders während der Nacht die Wahrscheinlichkeit der Bildung von Krebszellen reduziert.

Wichtig ist festzuhalten, dass dieser Inhibitions-Effekt gegen die Wirkung des Melatonins in Wechselfeldern von 50 bis 60 Hz am stärksten ist, während sie durch hochfrequente Wechselfelder im GHz-Bereich nicht verändert wird.

Zu erinnern ist daran, dass gemäss den europäischen Sicherheitsnormen elektromagnetische Felder bis 20'000 Milligauss als ungefährlich betrachtet werden, während die oben erwähnten Experimente zeigen, dass sich bereits bei 2 Milligauss Feldstärke eine spürbare Inhibitions-Wirkung gegen die Funktionen des Melatonins zeigt.

**RF-Schutzschild**

Im Folgenden ist zu zeigen, in welcher Weise eine Anordnung von Kohlefasern (Graphit), die im Gewebe "eingebaut" ist, einen Abschirmeffekt ausüben kann.

*Elektrostatische Felder*

Sicher ist uns allen schon passiert, dass wir beim Aussteigen aus dem Auto "einen Schlag" erhalten haben, wenn sich die elektrostatischen Ladungen, die sich beispielsweise infolge von Kriechströmen aus dem Elektroschaltkreis der Lichtmaschine angesammelt hatten, sich über unsern Körper geerdet und entladen haben.

Der gleiche Entladungsvorgang spielt sich in unserem Organismus in verschiedenster Weise ab, vor einem Fernsehschirm oder vor einem Computerbildschirm, oder vor einem Radioapparat, usw.

Ein Schutzschild aus Kohlefasern, der unseren Körper abdeckt, greift in wirksamer Weise ein, indem er die elektrischen Ladungen in den unzähligen Kondensatoren "einlagert", in die man sich das zufällig angeordnete "Fasernetzwerk" zerlegt als Idealmodell denken kann.

Mit andern Worten kann man sich dieses "Fasernetzwerk" als eine Anordnung von Kondensatoren mit ebenen Platten mit der Fläche $A_j$ vorstellen, was die Berechnungen erleichtert.

Mit "q" sei die Ladungsmenge des Kondensators mit den Platten $M_1$ und $M_2$ der Fläche "A" bezeichnet.

Im Fall mit isolierten Platten haben die Ladungen keinen Einfluss auf unseren Körper, da sie als im Kondensator "eingeschlossen" betrachtet werden können.

Wenn die beiden Platten über einen Widerstand R in Kontakt gebracht werden, entladen sie sich; während des Entladungsvorgangs entsteht ein Strom, der so lange fliesst, als die Entladung dauert:

$$i = dq/dt = MA \, dE/dt$$

wobei:

M = Dielektrizitätskonstante
E = Elektrisches Feld zwischen den Platten $M_1$ und $M_2$
A = Oberfläche der Kondensatorplatten
t =Zeit

Auch in diesem Fall wirkt sich der Entladungsstrom nicht auf unseren Körper aus, da sich die Energie $R_i{}^2$ auf den gleichen Graphit-Fasern abbaut, die zufälligerweise die beiden Platten des Kondensators in Kontakt gebracht haben. Anders ausgedrückt beeinflussen die elektrischen Felder, die wegen diesen Mikroströmen entstehen können, den durch ein solches Gewebe oder Schutztuch geschützten Organismus nicht.

*Elektromagnetische Felder*

Ohne auf die elektromagnetischen Wellen und deren Interpretation aufgrund der Maxwellschen Gleichungen einzugehen sei hier daran erinnert, was im Folgenden für das vorliegende Thema von Interesse ist:

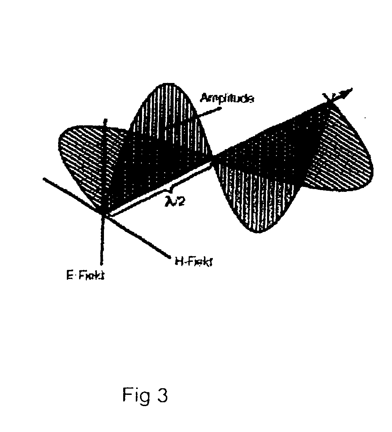

- Eine elektromagnetische Welle entsteht jedes Mal, wenn sich elektrische Ladungen in Bewegung befinden, und die Frequenz der Welle ist eine Funktion der Frequenz des elektrischen Stroms, der aus elektrischen Ladungen besteht, die sich in Bewegung befinden (Eine elektromagnetische Welle von 50 Hz wird also von einem Wechselstrom mit 50 Hz erzeugt). Die entsprechende Darstellung ist aus der Fig. 3 ersichtlich, in der sowohl die sinusförmige magnetische Welle als auch die sie erzeugende elektrische Welle dargestellt sind.

- Die einer elektromagnetischen Welle zugeordnete Energie wird durch den als "Pyontig-Vektor" bezeichneten Vektor dargestellt, der in die Richtung weist, in der sich die Energie fortpflanzt, und dessen Mass "EH" den der Welle zugeordneten Wert der Energie darstellt.

Daher ist klar, dass sich eine solche elektromagnetische Welle auf ihrem Weg weiter fortpflanzt, bis sie auf ein Hindernis stösst, das die ihr zugeordnete Energie aufnehmen kann, wie beispielsweise magnetisierbare Substanzen, in denen das magnetische Feld motorische Kräfte erzeugen kann oder in denen das elektrische Feld elektrische Ladungen bewegen kann und über den Durchgang solcher Ladungen durch einen elektrischen Widerstand aufzehren kann.

Generell lässt sich sagen, dass die Abnahme der elektromagnetischen Energie pro Zeiteinheit in einem geschlossenen Feld eine Fläche ergibt, die gleich ist wie der Fluss aus dem Poynting-Vektor, plus ein Glied, das der pro Zeiteinheit in Form von Wärme abgebauten Energie entspricht.

Offensichtlich ist dies bei unserem elektromagnetischen Schild der Fall, der aus Graphitfasern besteht, indem das elektrische Feld durch den Joule-Effekt in Form von Wärme abgebaut wird.

## Stand der Technik, Ungenügen der bisherigen Lösungen

Im Folgenden sind die aufgezeigten Probleme separat behandelt.

Die Konstrukteure haben jedes der zwei Probleme einzeln gelöst, in manchen Fällen sogar brillant, aber sie haben sich nicht zum Ziel gesetzt, die beiden Probleme in eindeutiger und harmonisch aufeinander abgestimmter Weise zu lösen; und genau dies ist das Ziel der vorliegenden Erfindung.

Es ist nicht müssig festzustellen, dass die gleichzeitige Lösung der beiden Probleme fast nie in einer banalen Summierung der beiden einzelnen Lösungen besteht, dass jedoch die separate Lösung des einen Problems Resultate ergibt, die der Lösung entgegensteht, die für das andere Problem erforderlich ist.

## Funktionsprinzip des erfindungsgemässen Gewebes und Überwindung der aufgezeigten Begrenzung gemäss dem beschriebenen Stand der Technik

Das Gewebe gemäss der vorliegenden Patentanmeldung stimmt die Lösungen harmonisch aufeinander ab, mittels welchen die Thematik der beschriebenen Punkte gleichzeitig zu überwinden ist.

Bei der Herstellung der Polymere (beispielsweise Polyester) wird während des Spinnprozesses zur Herstellung der Filamente Material eingebracht, sei es das biokeramische Material, das sich in der beschriebenen Weise auf die FIR auswirkt, oder seien es die Graphit-Elemente, welche die Abschirmung gegen die elektromagnetischen Felder aufbauen.

Die Wahl der einzubringenden Substanzen wird in solcher Weise getroffen, dass sie die nachher aufeinander folgenden industriellen Verarbeitungsprozesse der Filamente während des Spinnens, Webens (bzw. Herstellen des textilen Flächengebildes) und des Färbens bzw. Ausrüstens in keiner Weise beinträchtigen.

Wichtig ist zu unterstreichen, dass die Mischung bzw. der prozentuale Graphit-Anteil in solcher Weise gewählt wird, dass hauptsächlich die schädlichsten elektromagnetischen Felder abgewehrt werden, die wie vorgängig beschrieben jene sind, die mit Frequenzen von 50 bis 60 Hz schwingen.

Dieses Abwehrverhalten lässt sich leicht nachweisen mit Hilfe eines einfachen Detektorinstruments, wie es Elektriker verwenden, um die Lage elektrischer Unterputzleitungen in der Mauer festzustellen.

Wird dieses Instrument an einem Ort, wo es ein Feld anzeigt, mit einem Tuch aus irgendeinem Material, z. B. aus Baumwolle oder aus Acryl, zugedeckt, so zeigt das Instrument weiter das Vorhandensein des Feldes an, da die elektromagnetischen Wellen ungehindert durch das Tuch gehen; umgekehrt zeigt das Instrument, wenn es mit einem Gewebe gemäss der vorliegenden Erfindung zugedeckt wird, am gleichen Ort das Feld nicht mehr an, da in diesem Fall die elektromagnetische Abschirmung dazwischensteht.

**Patentansprüche**

1. Schutzgewebe bestehend aus mindestens zwei Fasertypen mit verschiedenen physikalischen Eigenschaften, **dadurch gekennzeichnet, dass** eine Komponente aus Fasern mit biokeramischem Material besteht, die zur Abstrahlung der vom menschlichen Körper ausgehenden Infrarotstrahlung dient, und eine Komponente aus Fasern besteht, die einen Abschirmeffekt gegen elektrostatische Felder und elektromagnetische Felder ausüben.

2. Schutzgewebe gemäss dem Anspruch 1, **dadurch gekennzeichnet, dass** die biokeramischen Materialien in den Fasern natürlichen Ursprungs sind, insbesondere Zeolite.

3. Schutzgewebe gemäss dem Anspruch 1, **dadurch gekennzeichnet, dass** die Fasern mit biokeramischem Material Infrarotstrahlen mit Wellenlängen im Wellenlängenbereich von 6 bis 14 Mikron ausstrahlen.

4. Schutzgewebe gemäss dem Anspruch 1, **dadurch gekennzeichnet, dass** das Strahlungsspektrum der biokeramischen Fasern jenem des schwarzen Körpers nahezu entspricht und eine Planck-Kurve der Leistungsabstrahlung zwischen 4 und 16 μm mit einem Maximum bei etwa 10 μm aufweist.

5. Schutzgewebe gemäss dem Anspruch 1, **dadurch gekennzeichnet, dass** die einen Abschirmeffekt gegen elektrostatische Felder und elektromagnetische Felder ausübenden Fasern Kohlefasern sind.

6. Anwendung des Schutzgewebes gemäss den Ansprüchen 1 bis 5, **dadurch gekennzeichnet, dass** das Gewebe zur Herstellung von Kleidungsstücken verwendet wird, insbesondere von Knieschonern, Ellbogenüberzügen, Leibbinden, Handgelenkstulpen, Trikots und Schutzabdeckungen für die am meisten der Strahlung ausgesetzten Körperteilen.

7. Anwendung des Schutzgewebes gemäss den Ansprüchen 1 bis 5, **dadurch gekennzeichnet, dass** das Gewebe verwendet wird zur Herstellung von Decken, Bettüberwürfen, Überwürfe sowie allgemein von Produkten die zum Zudecken von Personen während des Schlafs oder während des Ausruhens vorgesehen sind.

K I F A

# KOREA INSTITUTE OF FAR INFRARED APPLIED ESTIMATION
## KOREA FAR INFRARED ASSOCIATION

Add. : 174-12, Sokchon-Dong, Songpa-Ku, Seoul, Korea
Tel. : (02) 2203-6037,          FAX : (02) 2203-6061
Homepage : http://www.kfir.or.kr

## Certificate of Testing Result

Serial No. of Issue          : KFI-188

Name of Applicant          : Nam, Woo Hyun [B & R CORPORATION]

Address of Applicant      : #201, Youngsang B/D, 397-17, Daebang-Dong, Dongjak-Gu, Seoul, Korea

Date of Receipt              : 1. 31. 2002

Name of Test Sample      : Socks(Ceramic Treatment)

Result of Testing            : Refer to the Enclosed

| Emissivity<br>( 5 ~ 20 $\mu$m ) | Emission Power<br>(W/m$^2$·$\mu$m , 37℃) |
|---|---|
| 0.894 | 3.45 × 10$^2$ |

Note) The temperature of 37℃ is provided by the applicant.
The above experimental results were measured in comparison
with BLACK BODY by using the FT-IR Spectrometer. End.

| 2 | 5 | 2002 |
|---|---|---|
| month | day | year |

Signed *Choi T. S.*

The director of Korea Institute of
Far Infrared Applied Estimation

**Certificato della "Associazione Coreana del FAR Infrared"**
*(si nota che il campione ha un rendimento rispetto al "Corpo Nero" del 894 °/$_{oo}$ )*

fig 1/a

Figuren 1.a und 1b: Vergleich der Leistungsspektren des schwarzen Körpers

(obere Kurve) und des Mustergewebes (untere Kurve)

Fig. 2: Emissionskurve in Funktion der Wellenlänge bezogen auf den theoretischen Abstrahlungswert des schwarzen Körpers.

Fig 3

**Europäisches**
**Patentamt**

## EUROPÄISCHER RECHERCHENBERICHT

Nummer der Anmeldung

EP 04 40 5109

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | WO 01/88054 A (KOMURO TOSHIO) 22. November 2001 (2001-11-22) * Zusammenfassung * * Absätze [0002],[0006],[0011],[0020],[0034]-[0038] * --- | 1-4,6,7 | D03D15/00 A41D31/00 A61N5/00 |
| A | KR 2001 074 338 A (JUN BYUNG TEK ;CHA JUNG JIN (KR)) 4. August 2001 (2001-08-04) * Zusammenfassung * --- | 1-7 | |
| A | US 6 316 102 B1 (SASAKI KEIJI) 13. November 2001 (2001-11-13) * Zusammenfassung; Abbildung 3 * ----- | 1-4,6,7 | |

RECHERCHIERTE SACHGEBIETE (Int.Cl.7)

D03D
A41D
A61N

Der vorliegende Recherchenbericht wurde für alle Patentansprüche erstellt

| Recherchenort | Abschlußdatum der Recherche | Prüfer |
|---|---|---|
| MÜNCHEN | 6. Mai 2004 | Louter, P |

EPO FORM 1503 03.82 (P04C03)

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.......................................................................
& : Mitglied der gleichen Patentfamilie, übereinstimmendes Dokument

**ANHANG ZUM EUROPÄISCHEN RECHERCHENBERICHT
ÜBER DIE EUROPÄISCHE PATENTANMELDUNG NR.**

EP 04 40 5109

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-05-2004

| Im Recherchenbericht angeführtes Patentdokument | | Datum der Veröffentlichung | Mitglied(er) der Patentfamilie | | Datum der Veröffentlichung |
|---|---|---|---|---|---|
| WO 0188054 | A | 22-11-2001 | BR | 0110872 A | 11-02-2003 |
| | | | CA | 2406102 A1 | 22-10-2002 |
| | | | CN | 1426443 T | 25-06-2003 |
| | | | EP | 1291405 A1 | 12-03-2003 |
| | | | WO | 0188054 A1 | 22-11-2001 |
| KR 2001074338 | A | 04-08-2001 | KEINE | | |
| US 6316102 | B1 | 13-11-2001 | KEINE | | |

EPO FORM P0461

Für nähere Einzelheiten zu diesem Anhang : siehe Amtsblatt des Europäischen Patentamts, Nr.12/82